# EUROPEAN PATENT APPLICATION

(11) **EP 4 091 525 A1**
(43) Date of publication of application: **23.11.2022**
(21) Application number: 21174422.2
(22) Date of filing: 18.05.2021
(51) Int. Cl.: A61B 1/00, A61B 1/04, A61B 1/045, A61B 5/00

(54) **FLUORESCENCE TIMER**

(71) Applicant: Leica Instruments (Singapore) Pte. Ltd., Singapore 608924 (SG)
(72) Inventor: SCHWEIZER, Jochen, 86938 Schondorf am Ammersee (DE); EL HADDOUCHI, Hakim, 9436 Balgach (CH); SORMAZ, Milos, 8706 Meilen (CH)
(74) Representative: 2SPL Patentanwälte PartG mbB

(57) **Abstract**

A timer for a surgical imaging device is disclosed. The timer determines a working time of an imaging agent and outputs an indicator for display based on the working time. The working time can be a start time at which a signal from the agent is observable at an imaging site and/or an end time at which the agent is cleared.

## Description

### Technical field

Examples disclosed herein relate to a timer for surgical apparatuses such as imaging devices that use an indicator to provide contrast of anatomical features.

### Background

Modern surgical imaging devices that allow the visualization of anatomical features may utilize imaging agents, or probes, which may provide a contrast mechanism according to the imaging modality of the imaging device. Imaging agents that are introduced into a patient may have limited lifetimes, and or be cleared from the patient via various metabolic and/or other pathways. It can be challenging to utilize imaging agents effectively due to clearance and/or fading of the imaging agent signal over time, particularly when the time required for a surgical procedure is on the order of the working time of the imaging agent or longer.

### Summary

It may be desirable to be able to give notice to a surgeon of an upcoming reduction or loss of imaging signal. Such notice may allow for better decisions to be made in real-time during surgery. A surgeon may be able to pace the surgical procedure more appropriately to reduce patient trauma or other surgical risks, for example, because the surgeon is better informed about the working time of the imaging agent, e.g. when contrast from the imaging agent is adequate for image interpretation. A timer, a surgical microscope, and related methods of use are disclosed herein to address such issues.

Herein is disclosed a timer for a surgical imaging device, configured to determine a working time of an imaging agent and output an indicator for display, the indicator being based on the working time. An indicator of the working time of the imaging agent may inform a surgeon and enable adjustment of the pace of a surgical procedure more appropriately to reduce patient trauma or other surgical risks

The working time can be at least one of a start time (at which a signal from the agent may be observable at an imaging site), or an end time (at which the agent can be cleared). Providing a start and/or end time may allow a surgeon to adjust the pace of a procedure in order to take advantage of the best time window for performing the surgery.

The indicator can be a countdown timer showing at least one of a first duration until the start time or a second duration until the end time. A countdown timer may be well intuitively understood so that a surgeon can adjust the pace of a procedure in order to take advantage of the best time window for performing the surgery.

The working time can be based on at least one of: patient information, optical settings, instrumental parameters, dose of the imaging agent, agent administration time, agent administration type, or the imaging agent. Providing multiple factors for determination of the working time can increase the accuracy of the determination.

The imaging agent can be indocyanine green (ICG) or fluorescein sodium. ICG and/or fluorescein sodium may have working times on a time scale of a surgical procedure. An indication of the working time of such imaging agents can be particularly helpful to allow a surgeon to pace a procedure.

The timer can be configured such that: the working time is determined by multiple linear regression based on at least one of: a signal arrival time, a duration of signal, an end of signal time, patient information, optical settings, instrumental parameters, dose of the imaging agent, agent administration time, or agent administration type. Multiple parameter linear regression may be a particularly accurate way of determining the working time.

The timer can output a start recording signal for documenting a surgical procedure, the start recording signal being based on the working time, such as the start time thereof; or output an end recording signal, the end recording signal being based on the working time, such as the end time thereof. Having the timer provide the recordation start/stop signal may simplify operation of the device and/or may robustly provide records for surgical documentation.

The timer may output a control signal for controlling optical settings of the imaging device. The timer may be able to adjust optical settings so as to maximize the usable time-window of the imaging agent.

The timer may have at least one processor, and an output. A processor may efficiently make determinations about the working time. The output may allow for the indicator to be displayed and/or for control signals to be transmitted to a surgical device.

The timer may include a detector configured to detect the arrival of the imaging agent at an imaging site. The detector may allow the beginning of the working time to be determined. It may be useful for utilization of the beginning of the working time.

A surgical imaging device, as disclosed herein, can include the timer as described herein. The surgical imaging device can include a trigger to activate the timer or activate a recorder. The trigger can be operationally coupled to a port for administrating the imaging agent. The trigger can be user actuatable. The trigger can be activated by detection of fluorescence, e.g. at an imaging site. Providing a trigger to activate the timer can make use of the device easier. A recorder can be useful to enable review and/or documentation of procedures.

The surgical imaging device can include a display for displaying the indicator. The display can couple with an eyepiece of the surgical apparatus. A displayed timer can be useful for making the working time known to the user.

The surgical imaging device can be a near-infrared fluorescence microscope, such as one adapted or use with indocyanine green (ICG). The device can include a recorder for recording surgical procedures. A timer for use with ICG can be particularly useful because the clearance time for ICG can be a few minutes, or on about the same time scale as a surgical procedure, e.g. lasting not significantly longer than a surgical procedure. A timer for near-infrared microscopic imaging can be particularly helpful for providing intuitive information about the working time of fluorophores that may be completely invisible without the aid of extensive instrumentation.

A computer program comprising instructions to operate the timer is disclosed herein.

### Short Description of the Figures

Some examples of apparatuses and/or methods will be described in the following by way of example, and with reference to the accompanying figures, in which:
Fig. 1 illustrates a timer for a surgical device, according to embodiments described herein;
Fig. 2 illustrates a surgical imaging device, according to embodiments described herein;
Fig. 3 illustrates method of operating a surgical device, according to embodiments described herein;
Fig. 4 illustrates a block diagram for outputting an indicator based on a working time of an imaging agent, according to embodiments described herein; and
Fig. 5 illustrates a surgical imaging device, according to embodiments described herein.

### Detailed Description

Various examples will now be described more fully with reference to the accompanying drawings in which some examples are illustrated. In the figures, which are not to be assumed to be to scale, the thicknesses of lines, layers and/or regions may be exaggerated for clarity.

As used herein, the term "and/or" includes any and all combinations of one or more of the associated listed items and may be abbreviated as "/". Herein, a trailing "(s)" indicates one or more; for example processor(s) indicates one or more processors.

Herein, some aspects and/or technical features are described in the context of an apparatus. Technical features described in the context of the apparatus are also understood to describe a corresponding method, for example. Herein are disclosed methods of operating the apparatus, operating a surgical microscope, and operating a surgical device, for example. Aspects, steps, and/or technical features described in the context of a method also describe a corresponding technical feature of a corresponding apparatus. For example, determinations, as may be described with respect to a method and/or function, may be done by processor(s) of an apparatus. An apparatus described herein as performing a function or step within a method is a disclosure of performing the function and/or method as well.

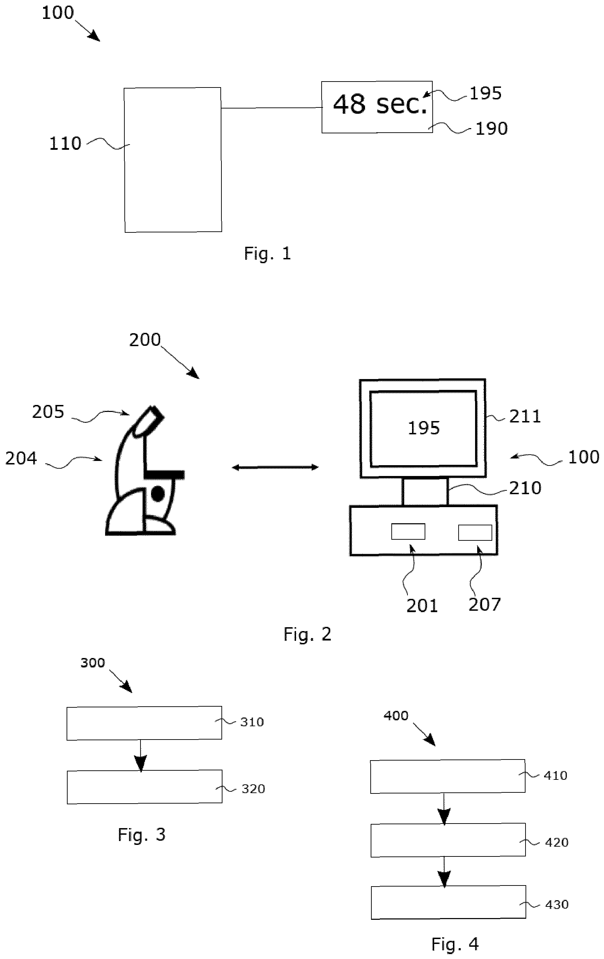

Fig. 1 illustrates a timer for a surgical device, according to embodiments described herein. The timer 100 can have at least one processor 110, such as a computer processor. The timer 100 determines a working time of an imaging agent and outputs an indicator 195. For example the output can be to a display 190, eyepiece, and/or ocular of a surgical imaging device. In the example of Fig. 1, the indicator 195 displays a working time *(e.g.* a remaining time of 48 seconds). The indicator 195 can be a countdown timer. Alternatively/additionally, an audible indicator is possible such as may include a beep or pattern of tones. An audible indicator may be useful, particularly at a point in time near the end of the useful time of the imaging agent, so that the surgeon is informed of upcoming loss of signal without having to remove his/her gaze. A displayed indicator 195 may also be helpful particularly if the indicator 195 is superimposed on an image of the surgical site, e.g. through ocular(s) or on-screen.

Fig. 2 illustrates a surgical imaging device 200. The device 200 has at least one processor 201. The device 200 may be coupled to microscope 204, such as a surgical microscope. The microscope 204 can be a near-infrared microscope, such as one adapted for use with ICG. The device can include a timer 100, as described herein. The device 200 can include an output 210 which can output image data for display. For example, the image data is transmitted to a display 211. The device 200 can include a display 211 for displaying the indicator 195. The display 211 can be, for example, a 2-D screen, a 3-D screen, or a digital viewer, and may include a touch sensitive screen for user input. Alternatively/additionally, the device can include an eyepiece 204 which can be used for displaying the indicator 195. The device 200 can include a memory 207, *e.g.* for storing recordings of surgical procedures.

The at least one processor 201 can be programmed for determining the working time, controlling optical settings of the imaging device 200, and/or outputting to the display 211. For example, the timer 100 described herein can include the least one processor 201.

Fig. 3 illustrates a method of operating a surgical device utilizing an imaging agent. The method 300 includes determining 310 a working time of the imaging agent and outputting 320 an indicator based on the working time. dd

Fig. 4 illustrates a block diagram for outputting an indicator based on the working time. The block diagram 400 shows various factors 410 which are used to determine a working time 420. An indicator 430 is output which is based on the working time 420. The factors 410 can include the amount of elapsed time from an event, such as from a trigger (e.g. administration of the imaging agent). The indicator 430 can be output by display and/or audible signal(s).

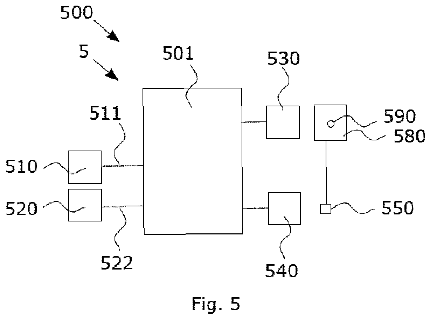

Fig. 5 illustrates a surgical imaging device 500. The device 500 at least one processor 501. The device 500 includes a timer 5 such as any in accordance with the timer described herein. The timer 5 and/or processor 501 can output a control signal 522 for controlling optical settings 520 of the device 500. For example, an imaging agent 580 is administered to a patient, such as through a port 550. A detector 530 at the imaging site 580 can detect the imaging agent 580. A trigger 540 can be activated, such as by the administration of the imaging agent 590, such as at the port 550. Alternatively/additionally, a trigger can be activated upon detection of the imaging agent 590 by the detector 530 at the imaging site 580.

A recorder 510 can be activated by detection of the imaging agent 590 by the detector 530 at the imaging site 580. The processor 501 and/or timer 5 can output a signal 511 for activating and/or deactivating the recorder 510. Control signals 522 can alternatively/additionally be output by the processor 501 and/or timer 5 to control optical settings 520 of the imaging device 500. The recorder 510 can include memory such as computer memory and /or storing media.

The determination of the working time may be based on any of a number of factors such as patient information, optical settings, instrumental parameters, dose of the imaging agent, agent administration time (*e.g.* time at which the imaging agent is administered to the patient, such as by injection), agent administration type (*e.g.* injection as a bolus or continually), and/or the imaging agent.

For example, the imaging agent can be fluorescein (*e.g.* fluorescein sodium) or indocyanine green (ICG). Different imaging agents may be cleared at different rates from a patient. The determination of the working time of the imaging agent can take into account the identity of the imaging agent (*e.g.* its clearance rates and/or properties). The timer 100 may be particularly useful for fluorescent imaging agents such as ICG, which may have clearance half-lives on the order of 1-2 minutes or so.

As an example, ICG can be administered (such as injected into a patient) and used as a contrast agent for infrared imaging. Injection may trigger countdown of the indicator, *e.g.* countdown until a fluorescence signal is expected at the imaging and/or surgical site. After administration of the ICG, the infrared fluorescence signal appears, *e.g.* after the ICG circulates to the surgical site. Over time, as ICG is subsequently cleared, the infrared fluorescence signal for the fluorescence image may become weaker, e.g. as the imaging agent is cleared. For example, over time, the concentration of ICG in the blood is reduced, such that the fluorescence becomes weaker over time.

In a surgical procedure, it is desirable to have information as to when the signal from the imaging agent is lost. An indicator which is based on the working time of the imaging agent may provide such information. The working time can include an end time at which the signal from the agent is cleared and//or the signal is unobservable at a surgical site. The end time can be determined based on the patient information, optical settings, instrumental parameters, dose of the imaging agent, agent administration time (*e.g.* time at which the imaging agent is administered to the patient, such as the injection time), or agent administration type (such as if the administration is to inject a bolus or if the administration is perfused over time), and/or the imaging agent (whether the imaging agent is indocyanine green or fluorescein sodium, for example). Additional/alternative imaging agents can also be used. The fluorescence timer 100 can be adapted to imaging agents that have different working times.

It may also be desirable to have information as to when the signal turns on. For example, a duration of time may pass while the imaging agent travels from the point of introduction (*e.g.* an injection site in the arm) to the imaging site, *e.g.* via the circulatory system of the patient. The working time of an imaging agent can include a start time, such as a time at which the signal from the agent is observable at a surgical intervention site (or expected to be observable at the site). The timer 100 can include a detector which detects the arrival of the imaging agent at the imaging site. For example, the trigger is activated by detecting fluorescence at the imaging site.

Alternatively/additionally, the timer 100 determines and applies optical settings, *e.g.* by outputting control signals, based on the trigger. For example, once a fluorescence imaging agent is administered, the start time is determined, and the timer controls the excitation source, *e.g.* powering the excitation source at the start time (or immediately before the start time), which may coincide with the time that the imaging agent reaches the imaging site and/or becomes detectable.

A trigger can be coupled to the timer. For example, a trigger can activate the timer. The trigger can alternatively/additionally be operationally coupled to a detector, *e.g.* such that when the imaging agent is detected at the imaging site and/or site of administration, the trigger is activated. Alternatively/additionally, the trigger is operationally coupled to a port for administrating the imaging agent, *e.g.* such that when the imaging agent is administered, the trigger is activated. Alternatively/additionally, the trigger is activated by the user.

Having a detector at the imaging site which triggers the timer 100 and/or recording device may be advantageous in that detection of the imaging agent arrival at the imaging site can reduce uncertainty in the determination of the end time and/or the surgeon can utilize the initial usable working time of the imaging agent. Having a trigger coupled to a port may be useful for estimating the start time in advance, e.g. may provide for a useful countdown timer before the imaging agent is expected at the imaging site. A user activated trigger may alternatively/additionally be useful for *ad hoc* adjustments to working time determinations, and/or for adjustments for machine learning of the working time.

The timer 100 can be coupled to a recorder, such as for documenting the surgical procedure. Alternatively/additionally, the timer 100 may include the recorder. The timer 100 can output a start recording signal which is determined based on the working time such as the start time thereof. The timer 100 and/or trigger thereof can initiate the recorder and/or the start recording signal. The timer 100 may also output an end recording signal, *e.g.* an end recording signal based on the working time, such as the end time thereof. For example, the duration of the recording is determined by at least one of the working time, instrument settings, the exposure time (*e.g.* fluorescence signal integration time), excitation source power, detector gain(s), or patient information. The recorder may use computer memory for storing the recording.

The trigger may be a start button on a user interface, such as a touch sensitive screen, *e.g.* a touch sensitive screen that can show the output of the timer. For example, when the imaging agent is injected, the trigger is activated, and the timer 100 counts down to when the imaging signal will be visible (and may also trigger recording).

The timer 100 may be operationally coupled to an imaging device.

For example, the timer can output control signal(s) for controlling optical settings of the imaging device. Optical settings that may be controlled include at least one of: turning on/off an excitation source, adjust brightness of the source, detector gain settings, selection of filters, or adjust exposure time. For example, the timer may send control signals to compensate for imaging agent clearance. For example, as a fluorescent imaging agent is cleared, the brightness of the excitation source can be increased, the exposure time can be increased, and/or the detector gains can be increased. These examples of countermeasures may compensate for the reduction in imaging agent concentration (*e.g.* clearance of the fluorescent imaging agent) which would normally result in reduction of signal. It may be possible, to an extent, to lengthen the effective working time of the imaging agent by controlling optical settings of the imaging device.

The start time can be based on patient information, optical settings, instrumental parameters, dose of the imaging agent *(e.g.* in mass, volume, and/or concentration), agent administration time (*e.g.* time at which the imaging agent is administered to the patient, such as by injection), or agent administration type, and/or the imaging agent.

For example, the working time of the imaging agent can include a start time, an end time, and/or a duration of effectiveness which can be the difference between the start and end times.

The indicator 195 can be a countdown timer that shows a duration of time remaining until an event, such as until the end time is reached or until the start time is reached.

The determination of the working time may be influenced by multiple parameters. A linear regression model may be used to determine the relationship between the parameters and the working time. For example, a multiple linear regression model(s) for working time *(e.g.* signal arrival time and/or duration of signal) can be determined based on at least one of : a signal arrival time, a duration of signal, an end of signal time, patient information, optical settings, instrumental parameters, dose of the imaging agent, agent administration time, or agent administration type.

Machine learning may be used to determine the working time. Machine learning can refer to algorithms and/or statistical models that computer systems may use to perform task(s) possibly without using particularized explicit instructions, instead relying on models and inference. For example, in machine-learning, instead of a rule-based transformation of data, a transformation of data may be used, that is inferred from an analysis of historical and/or training data. For example, data (which can include multiple factors as described herein including determined working times) may be analyzed using a machine-learning model or using a machine-learning algorithm.

The machine-learning model may be trained using training parameters as input and working times as output. By training the machine-learning model with a large number of training working times and associated training information (factors and/or parameters like imaging agent, patient information, optical settings, instrumental parameters, dose of the imaging agent, agent administration time, and/or agent administration type), the machine-learning model can learn to recognize the training information. Working times can be determined based on the machine-learning model. The same learning principle may be used for multiple factors and/or parameters that can affect working time.

By training a machine-learning model using training data and working times, the machine-learning model can learn a transformation between the factors and the working time, which can be used to provide an output based on non-training data provided to the machine-learning model.

Machine-learning models can be trained using training input data. The examples specified above may use supervised learning. In supervised learning, the machine-learning model can be trained using a plurality of training samples. Each training sample may comprise a plurality of input data values, and a plurality of desired output values, *i.e.* each training sample is associated with a desired output value. By specifying both training samples and desired output values, the machine-learning model learns which output value to provide based on an input sample that is similar to the samples provided during the training.

Machine learning may be used to determine the relations between predictors (such as parameters such as imaging agent, patient information, imaging agent dose, optical settings, instrumental parameters, administration type, *etc*.) and the working time. Particularly, one or more regression algorithms may be used, such as linear regression, ordinary least squares regression, stepwise regression, multivariate adaptive regression splines, locally estimated scatterplot smoothing, and/or logistic regression. Alternatively/additionally, regularization can be used, such as ridge regression.

Some embodiments relate to a microscope comprising a system as described in connection with one or more of the figures. Alternatively/additionally, a microscope may be part of or connected to a system as described in connection with one or more of the figures.

A surgical imaging device 200 can be configured to perform a method described herein. The The device 200 can be configured to execute at least a part of a method described herein. The computer system, such as the processor(s) 201 thereof, may be configured to execute a machine learning algorithm, as described herein. The computer system and microscope may be separate entities but can also be integrated together in one common housing. The computer system may be part of a central processing system of the microscope and/or the computer system may be part of a subcomponent of the microscope, such as a sensor, an actor, a camera or an illumination unit, etc. of the microscope.

The computer system may be a local computer device (e.g. personal computer, laptop, tablet computer or mobile phone) with one or more processors and one or more storage devices or may be a distributed computer system (e.g. a cloud computing system with one or more processors and one or more storage devices distributed at various locations, for example, at a local client and/or one or more remote server farms and/or data centers). The computer system may comprise any circuit or combination of circuits. In one embodiment, the computer system may include one or more processors which can be of any type. As used herein, processor may mean any type of computational circuit, such as but not limited to a microprocessor, a microcontroller, a complex instruction set computing (CISC) microprocessor, a reduced instruction set computing (RISC) microprocessor, a very long instruction word (VLIW) microprocessor, a graphics processor, a digital signal processor (DSP), multiple core processor, a field programmable gate array (FPGA), for example, of a microscope or a microscope component (e.g. camera) or any other type of processor or processing circuit. Other types of circuits that may be included in the computer system may be a custom circuit, an application-specific integrated circuit (ASIC), or the like, such as, for example, one or more circuits (such as a communication circuit) for use in wireless devices like mobile telephones, tablet computers, laptop computers, two-way radios, and similar electronic systems. The computer system may include one or more storage devices, which may include one or more memory elements suitable to the particular application, such as a main memory in the form of random access memory (RAM), one or more hard drives, and/or one or more drives that handle removable media such as compact disks (CD), flash memory cards, digital video disk (DVD), and the like. The computer system may also include a display device, one or more speakers, and a keyboard and/or controller, which can include a mouse, trackball, touch screen, voice-recognition device, or any other device that permits a system user to input information into and receive information from the computer system.

Some or all of the method steps may be executed by (or using) a hardware apparatus, like for example, a processor, a microprocessor, a programmable computer or an electronic circuit. In some embodiments, some one or more of the most important method steps may be executed by such an apparatus.

Depending on certain implementation requirements, embodiments of the invention can be implemented in hardware or in software. The implementation can be performed using a non-transitory storage medium such as a digital storage medium, for example a HDD (hard disk drive), an SSD (solid state drive), a floppy disc, a DVD, a Blu-Ray, a CD, a ROM, a PROM, and EPROM, an EEPROM or a FLASH memory, having electronically readable control signals stored thereon, which cooperate (or are capable of cooperating) with a programmable computer system such that the respective method is performed. Therefore, the digital storage medium may be computer readable.

Some embodiments according to the invention comprise a data carrier having electronically readable control signals, which are capable of cooperating with a programmable computer system, such that one of the methods described herein is performed.

Generally, embodiments of the present invention can be implemented as a computer program product with a program code, the program code being operative for performing one of the methods when the computer program product runs on a computer. The program code may, for example, be stored on a machine readable carrier.

Other embodiments comprise the computer program for performing one of the methods described herein, stored on a machine readable carrier.

In other words, an embodiment of the present invention is, therefore, a computer program having a program code for performing one of the methods described herein, when the computer program runs on a computer.

A further embodiment of the present invention is a storage medium (or a data carrier, or a computer-readable medium) comprising, stored thereon, the computer program for performing one of the methods described herein when it is performed by a processor. The data carrier, the digital storage medium or the recorded medium are typically tangible and/or non-transitionary. A further embodiment of the present invention is an apparatus as described herein comprising a processor and the storage medium.

A further embodiment of the invention is, therefore, a data stream or a sequence of signals representing the computer program for performing one of the methods described herein. The data stream or the sequence of signals may, for example, be configured to be transferred via a data communication connection, for example, via the internet.

A further embodiment comprises a processing means, for example, a computer or a programmable logic device, configured to, or adapted to, perform one of the methods described herein.

A further embodiment comprises a computer having installed thereon the computer program for performing one of the methods described herein.

A further embodiment according to the invention comprises an apparatus or a system configured to transfer (for example, electronically or optically) a computer program for performing one of the methods described herein to a receiver. The receiver may, for example, be a computer, a mobile device, a memory device or the like. The apparatus or system may, for example, comprise a file server for transferring the computer program to the receiver.

In some embodiments, a programmable logic device (for example, a field programmable gate array) may be used to perform some or all of the functionalities of the methods described herein. In some embodiments, a field programmable gate array may cooperate with a microprocessor in order to perform one of the methods described herein. Generally, the methods are preferably performed by any hardware apparatus.

Herein the patient information that can be used as a basis for determining working time of the imaging agent can include weight, sex, blood pressure, heart rate, breathing rate, and/or drug usage. Patient information may or may not include real-time patient information such as real-time heart rate, breathing rate, and/or blood pressure.

Herein, "imaging site," "surgical site," and "surgical intervention site" may be used interchangeably.

Herein, an imaging agent can be "cleared" when the signal from the imaging agent at the imaging site is reduced below a threshold, such as a threshold determinable from a baseline and/or noise floor.

The examples disclosed hereinabove are illustrative and not intended to be limiting. Reference numerals are given to aid in the understanding of the invention, and are for illustrative purposes, and not intended to be limiting. The invention is defined by the appended claims and their equivalents.

**List of reference signs**

| | |
|---|---|
| 100 | timer |
| 110 | processor |
| 190 | display |
| 195 | indicator |
| 200 | surgical imaging device |
| 201 | processor |
| 204 | microscope |
| 205 | eyepiece |
| 207 | memory |
| 210 | output |
| 211 | display |
| 300 | method |
| 310 | determining a working time |
| 320 | outputting an indicator |
| 400 | block diagram |
| 410 | factors |
| 420 | working time |
| 430 | indicator |
| 501 | processor |
| 510 | recorder |
| 511 | recording signal |
| 520 | optical settings |
| 522 | control signal |
| 530 | detector |
| 540 | trigger |
| 550 | port |
| 580 | imaging site |
| 590 | imaging agent |

## Claims

1. A timer (100) for a surgical imaging device (200, 500),
configured to:
determine a working time (420) of an imaging agent (590); and
output an indicator (195) for display based on the working time (420).

2. The timer (100) of any preceding claim, wherein
the working time (420) is at least one of:
a start time at which a signal from the imaging agent (590) is observable at an imaging site, or an end time at which the imaging agent (590) is cleared.

3. The timer (100) of claim 2, wherein
the indicator (195) is a countdown timer showing at least one of a first duration until the start time or a second duration until the end time.

4. The timer (100) of any preceding claim, wherein
the working time (420) is based on at least one of:
patient information, optical settings, instrumental parameters, dose of the imaging agent, agent administration time, agent administration type, or the imaging agent.

5. The timer (100) of claim 4, wherein
the imaging agent (590) is indocyanine green or fluorescein sodium.

6. The timer (100) of any preceding claim, wherein the timer (100) is configured such that:
the working time (420) is determined by multiple linear regression based on at least one of: a signal arrival time, a duration of signal, an end of signal time, patient information, optical settings, instrumental parameters, dose of the imaging agent, agent administration time, or agent administration type.

7. The timer (100) of any preceding claim, further configured to at least one of:
output a start recording signal (511) for documenting a surgical procedure, the start recording signal (511) being based on a start time of the working time (420); or
output an end recording signal (511), the end recording signal (511) being based on an end time of the working time (420).

8. The timer (100) of any preceding claim, further configured to:
output a control signal (522) for controlling optical settings (520) of an imaging device (500).

9. The timer (100) of any preceding claim, further comprising
at least one processor (201), and
an output (210).

10. The timer (100) of any preceding claim, further comprising
a detector (530) configured to detect an arrival of the imaging agent (590) at an imaging site (580).

11. The timer (100) of any preceding claim, further configured to determine the working time by a machine learning algorithm.

12. A surgical imaging device (200, 500), comprising
the timer (100) of any of claims 1-11, and
a trigger (540) to activate the timer (100) or activate a recorder,
the trigger (540) being at least one of: operationally coupled to a port (550) for administrating the imaging agent (590), user actuatable, or actuatable by detection of fluorescence at an imaging site (580).

13. The surgical imaging device (200, 500) of claim 11 or 12, further comprising
a display (190) for displaying the indicator (195), the display couplable with an eyepiece (205) of the surgical imaging device (200).

14. The surgical imaging device (200, 500) of claim 11 or 12, wherein
the device is a near-infrared fluorescence microscope, comprising a recorder (520) for recording a surgical procedure.

15. A computer program comprising instructions to operate the timer (100) of any of claims 1-14.
